# EUROPÄISCHE PATENTSCHRIFT

(11) **EP 3 189 770 B1**
(45) Veröffentlichungstag und Bekanntmachung des Hinweises auf die Patenterteilung: **05.01.2022**
(21) Anmeldenummer: 17157622.6
(22) Anmeldetag: 07.11.2011
(51) Int. Cl.: A61B 1/00, B23K 1/19

(54) **VERFAHREN ZUM MONTIEREN EINES DECKGLASES IN EINEM ENDOSKOP**
METHOD FOR FITTING A GLASS COVER IN AN ENDOSCOPE
PROCÉDÉ DE MONTAGE D'UNE LAMELLE COUVRE-OBJET DANS UN ENDOSCOPE

(30) Priorität: 08.11.2010 DE 102010050513
(43) Veröffentlichungstag der Anmeldung: 12.07.2017
(62) Teilanmeldung aus: 11188076.1
(73) Patentinhaber: Karl Storz SE & Co. KG, 78532 Tuttlingen (DE)
(72) Erfinder: Eisenkolb, Peter, 78532 Tuttlingen (DE); Heseler, Stefan, 78532 Tuttlingen (DE); Lehmann, Martin, 78357 Zoznegg (DE)
(74) Vertreter: Patent- und Rechtsanwälte Behrmann Wagner PartG mbB

(56) Entgegenhaltungen:
- DE-A1- 10 344 109
- DE-T2- 69 813 119
- DE-U1- 9 309 545
- US-A1- 2008 045 991

## Beschreibung

Die vorliegende Erfindung betrifft ein Verfahren zum Montieren eines Deckglases in einem Endoskop.

Ein Endoskop umfasst typischerweise einen lang erstreckten Schaft, der zur Einführung in einen Hohlraum geeignet ist, sowie einen Kopf, der Anschlüsse und Bedienelemente sowie einen Okulareinblick aufweisen kann. Zusätzlich oder anstelle des Okulareinblicks kann ein Anschluss für eine Videokamera vorhanden sein; häufig ist das Okular selbst für den Anschluss einer Videokamera ausgebildet, die beispielsweise an einer Okularmuschel befestigt werden kann. Innerhalb des Schafts und des Kopfs ist ein optisches System zur Weiterleitung eines endoskopischen Bildes vom distalen, d.h. beobachterfernen, Ende des Endoskops zum proximalen, d.h. beobachternahen, Ende angeordnet. Das optische System kann hierfür insbesondere ein distal angeordnetes Objektiv zur Aufnahme des endoskopischen Bilds, einen Bildweiterleiter und ein Okular mit einem Okulareinblick zur visuellen Betrachtung des weitergeleiteten endoskopischen Bildes umfassen. Das Endoskop kann insbesondere mit einem starren Schaft ausgebildet sein und als Bildweiterleiter eine Stablinsenanordnung aufweisen, kann jedoch auch semiflexibel oder flexibel mit einem Glasfaserbündel als Bildweiterleiter ausgebildet sein. Das Endoskop kann zur Beleuchtung eines zu beobachtenden Hohlraums eine Beleuchtungseinrichtung aufweisen sowie ggf. Kanäle für endoskopische Arbeitsinstrumente.

Aus hygienischen Gründen müssen für medizinische Zwecke vorgesehene Endoskope sterilisierbar sein. Die Sterilisation erfolgt heute zumeist durch Autoklavieren, d.h., durch heißen Dampf bei erhöhtem Druck, beispielsweise bei 2 bar und einer Temperatur von ca. 140 °C. Optische Systeme von Endoskopen sind jedoch in der Regel empfindlich gegen heißen Dampf, der bei der Heißdampfsterilisation eindringen und beispielsweise zu Glaskorrosion führen kann. Darüber hinaus kann in ein optisches System eingedrungene Feuchtigkeit zum Beschlagen optischer Elemente führen und dadurch die Funktion beeinträchtigen. Deshalb sind Schutzmaßnahmen gegen das Eindringen von Heißdampf, insbesondere unter den bei der Heißdampfsterilisation verwendeten Druckbedingungen, erforderlich. Diese Schutzmaßnahmen sind auch bei anderen Sterilisationsmethoden, wie etwa der chemischen Sterilisation, sowie beispielsweise bei technischen Anwendungen in Hohlräumen, die aggressive gasförmige oder flüssige Medien enthalten, vorteilhaft.

Insbesondere kann das optische System in einem dampfdichten Rohr aufgenommen sein, das durchgehend ausgeführt ist oder aus einer Mehrzahl von Rohren bzw. Hülsen, die dampfdicht miteinander verbunden sind, besteht. Das proximale und das distale Ende des Rohrs sind dabei jeweils durch Deckgläser verschlossen, die dampfdicht mit dem Rohr bzw. einer mit dem Rohr verbundenen Hülse verbunden sind.

Zum Herstellen einer dampfdichten Verbindung zwischen einem Deckglas und dem Rohr bzw. einer Hülse ist es bekannt, das Deckglas mit dem Rohr bzw. der Hülse zu verkleben, beispielsweise durch Umspritzen des Deckglases mit einem Kunststoff, beispielsweise PPSU (Polyphenylsulfon), aus dem auch beispielsweise die Okularmuschel bestehen kann. PPSU ist jedoch nicht ausreichend widerstandsfähig gegenüber einigen bei der Desinfektion und Sterilisation verwendeten Chemikalien und kann sich bei der Aufbereitung verfärben. Auch andere Kunststoffe bzw. Klebstoffe haben Nachteile hinsichtlich der Widerstandsfähigkeit bei der Sterilisation und können nicht immer eine reproduzierbar dichte Verbindung gewährleisten Verbindung gewährleisten beziehungsweise können Diffusionsvorgänge von Feuchtigkeit durch den Kunststoff bzw. Klebstoffe nicht ausreichend verhindern oder eindämmen.

Aus DE 37 40 417 Al ist es bekannt, bei einer starren Endoskopoptik mit einem die Linsen aufnehmenden Metallrohr, das am distalen Ende mit einem Fenster verschlossen ist, die Umfangsfläche des distalen Endfensters mit einen Metallfilm zu beschichten und diesen Metallfilm mit der Rohrinnenfläche zu verlöten.

Gemäß WO 98/04948 wird ein Endfenster eines Endoskops an seinem Umfangsrand in ein Außenrohr, innerhalb dessen das optische System aufgenommen ist, oder in ein in das Au-ßenrohr eingesetztes Zwischenstück eingeklebt, wobei das Endfenster zusätzlich durch eine Lotschicht mit dem Außenrohr oder dem Zwischenstück verbunden ist. Hierfür ist auf wenigstens einer Endfläche des Endfensters eine lötbare ringförmige Schicht aufgebracht.

Das Aufbringen einer solchen lötfähigen metallischen Beschichtung stellt jedoch einen zusätzlichen Aufwand dar. Es wäre deshalb wünschenswert, die Verlötung nicht mit einem zuvor aufgebrachten Metallfilm vorzunehmen, sondern direkt mit dem Glas, was hier in der Regel Saphirglas ist. Ein aufwandsgünstiges Verfahren zum Löten von Saphirglas ist das Aktivlöten, bei dem bei einer Temperatur von ca. 900 °C oder mehr im Vakuum ein spezielles Lot, das insbesondere Silber, Kupfer und Titan enthalten kann, aufgebracht wird. Hierbei ist keine vorherige Beschichtung mit einer lötfähigen Schicht erforderlich. Bei diesen Bedingungen wird jedoch eine Antireflexbeschichtung, die zur Verbesserung der Bildqualität zumindest auf der Innenseite des Deckglases vorteilhaft ist, zerstört. Eine solche Antireflexbeschichtung selbst ist nicht lötfähig. In US 5,536,244 oder der DE 93 09 545 U1 ist offenbart, dass ein Endfenster direkt in einen metallischen Rahmen eingelötet wird, der in den Endoskopschaft eingesetzt und mit diesem verschweißt wird; eine Beschichtung ist nicht erwähnt.

Die US 2008/045991 A1 zeigt ein Verfahren, wobei ein langgestrecktes zylindrisches Keramikelement mit einer metallisierten Oberfläche, insbesondere ein Saphirglaselement, in ein rohrförmiges Metallelement gefügt wird und anschließend in einzelne Deckgläser mit Metallringen geschnitten wird. Nach einer polierenden Oberflächenbehandlung des geschnittenen Deckglases kann eine Antireflexionsbeschichtung aufgebracht werden.

Die DE 698 13 119 T2 offenbart ein optisches Beobachtungssystem, insbesondere eine Handkamera, wobei auf einer äußeren Okularoberfläche, das zu dem Auge eines Betrachters am nächsten liegt, eine Antibeschlagbeschichtung ausgebildet ist.

Es ist die Aufgabe der vorliegenden Erfindung, ein Verfahren zum Montieren eines Deckglases in einem Endoskop, wobei die o.g. Nachteile vermieden werden. Insbesondere ist es die Aufgabe der vorliegenden Erfindung, ein vereinfachtes Verfahren anzugeben, mit dem ein Endoskop mit einem eingelöteten Deckglas erhalten wird, das eine Beschichtung, insbesondere eine Antireflexbeschichtung, aufweist. Bei der Beschichtung könnte es sich alternativ beispielsweise auch um eine Anti-Fog-Beschichtung handeln.

Diese Aufgabe wird durch ein Verfahren mit den Merkmalen der unabhängigen Ansprüche gelöst.

Bei einem erfindungsgemäßen Verfahren zum Montieren eines Deckglases in einem Endoskop wird zunächst ein unbeschichtetes Deckglas in einen Zwischenring eingelötet. Das Deckglas weist in diesem Zustand insbesondere keine metallische Beschichtung auf. Im nächsten Schritt wird das in den Zwischenring eingelötete Deckglas mit einer Beschichtung versehen, insbesondere mit einer Beschichtung, die zur Verbesserung der optischen Eigenschaften des Deckglases bzw. des optischen Systems des Endoskops dient. Hierbei kann sich ein Teil der Beschichtung auf dem Zwischenring niederschlagen, was dessen Verwendung jedoch nicht beeinträchtigt. In einem weiteren Schritt wird schließlich der Zwischenring, der das mit der Beschichtung versehene Deckglas trägt, in eine Hülse des Endoskops gefügt. Die Hülse kann als Rohr oder als Teil des Rohrs ausgebildet sein, das das optische System des Endoskops zumindest teilweise umschließt oder kann mit dem Rohr verbunden sein. Insbesondere verläuft der Strahlengang des Endoskops durch die Hülse, und das Deckglas kann als distales Endfenster, d.h. als Eintrittsfenster, oder als proximales Endfenster, d.h. als Austrittsfenster des Strahlengangs bzw. des endoskopischen Bilds ausgebildet sein. Das Deckglas besteht insbesondere aus Saphirglas.

Dadurch, dass das Deckglas in den Zwischenring eingelötet wird, der in die Hülse des optischen Systems des Endoskops gefügt wird, kann eine gas- und fluiddichte, d.h. dampfdichte, insbesondere eine hermetisch dichte Verbindung zwischen dem Deckglas und der Hülse und damit ein dampfdichter bzw. ein hermetisch dichter Abschluss des optischen Systems erreicht werden. Darüber hinaus wird dadurch, dass das Deckglas zunächst im unbeschichteten Zustand in den Zwischenring eingelötet wird und die Beschichtung nach diesem Schritt aufgebracht wird, vermieden, dass die Beschichtung durch den Lötvorgang beschädigt wird, beispielsweise durch die mechanische Behandlung, die thermischen und/oder die chemischen Bedingungen beim Löten. Schließlich wird dadurch, dass die Beschichtung auf das in den Zwischenring eingelötete Deckglas aufgebracht wird, der Beschichtungsvorgang erleichtert und die Qualität der Beschichtung verbessert. Insbesondere wird hierdurch das beim Beschichten eines in die Hülse eingesetzten Deckglases auftretende Problem vermieden, dass eine Beschichtung nicht oder nicht in ausreichender Qualität innerhalb der Hülse auf die Innenseite des Deckglases aufgebracht werden kann. Dies kommt dadurch zustande, dass die Hülse typischerweise um eine Mehrfaches des Durchmessers des Deckglases über dessen innere Oberfläche vorsteht. Demgegenüber ragt der Zwischenring nicht oder nur um einen Bruchteil des Durchmessers des Deckglases in axialer Richtung über die innere Oberfläche hinaus. Die zu beschichtende Oberfläche des Deckglases wird daher durch den Zwischenring beim Beschichtungsvorgang nicht in erheblichem Maße abgeschattet und der Beschichtungsvorgang nicht wesentlich beeinträchtigt. Das Beschichten kann beispielsweise durch Aufdampfen erfolgen.

In bevorzugter Weise wird das Deckglas durch Hartlöten, insbesondere durch Aktivlöten, in den Zwischenring eingelötet. Hierdurch kann eine sichere, temperatur- und chemisch beständige, dampfdichte Verbindung zwischen dem Deckglas und dem Zwischenring erreicht werden. Ferner werden hierdurch geringere Spannungen auf das Deckglas ausgeübt als bei anderen Lötverfahren. Erfindungsgemäß ist der Einsatz des Hartlötens bzw. des Aktivlötens nicht durch eine auf dem Deckglas bereits vorhandene Beschichtung eingeschränkt. Insbesondere kann durch Aktivlöten eine sichere Verbindung erreicht werden, ohne dass zuvor eine lötfähige Schicht auf das Deckglas aufgebracht werden müsste. Hierfür ist Saphirglas besonders geeignet.

Gemäß einer bevorzugten Ausführungsform der Erfindung wird das Deckglas dadurch in den Zwischenring eingelötet, dass zunächst das Deckglas in den Zwischenring eingesetzt wird, wobei ein Spalt zwischen einer insbesondere zylindrischen Umfangsfläche des Deckglases und einer insbesondere ebenfalls zylindrischen Innenfläche des Zwischenrings verbleibt. Beim darauffolgenden Lötvorgang dringt das Lot in den Spalt ein und füllt diesen zumindest teilweise aus. Hierdurch kann auf besonders einfache Weise eine besonders sichere und feste Verbindung zwischen Deckglas und Zwischenring erzielt werden. Dabei kann eine dampfdichte Verbindung insbesondere dann mit größtmöglicher Sicherheit erreicht werden, wenn die Höhe des Spalts ausreichend groß ist, so dass ggf. verbleibende Poren von Lot umschlossen werden und keine Undichtigkeit verursachen.

Weiterhin ist es vorteilhaft, wenn das Lot zwischen einer Umfangsfläche des Deckglases und einer ringförmigen Planfläche des Zwischenrings eine Kehlnaht bildet. Hierdurch wird eine zusätzliche Sicherheit zur Erzielung einer festen und dampfdichten Verbindung gewährleistet.

Erfindungsgemäß wird die Beschichtung zumindest auf die Innenseite des Deckglases aufgebracht. Eine Beschichtung kann auch auf die Außenseite des Deckglases aufgebracht werden, doch diese kann bei den beim Gebrauch des Endoskops wiederholt notwendigen Reinigungs- und Sterilisationsmaßnahmen beschädigt werden und hat daher in der Regel einen geringeren Nutzen als eine innenseitige Beschichtung. Sofern sich neben der Oberfläche des Deckglases auch ein Teil der Beschichtung auf dem Zwischenring ablagert, hat dies keine nachteiligen Konsequenzen.

Gemäß einer besonders bevorzugten Ausführungsform der Erfindung ist die Beschichtung eine Antireflexschicht. Eine solche Antireflexschicht verbessert die Transmission und verringert unerwünschte Reflexionen innerhalb des Endoskops. Erfindungsgemäß sind die Aufbringung und die Art der Antireflexschicht nicht durch die Bedingungen des Lötvorgangs eingeschränkt.

Alternativ könnte die Beschichtung auch eine Antibeschlagschicht (Anti-Fog-Schicht) sein.

Vor dem Fügen des Zwischenrings in die Hülse kann in vorteilhafter Weise ein Trockenmittel in die Hülse eingebracht werden. In dieser Hinsicht wird Bezug genommen auf die Patente DE 10344109 B4 und EP 1370175 B1, deren Inhalt hiermit in diese Anmeldung aufgenommen wird. Durch Einbringen eines Trockenmittels, beispielsweise nach der Lehre eines der referenzierten Patente, kann eine höchstmögliche Sicherheit gegen ein Beschlagen der Elemente des optischen Systems erreicht werden, wenn Restfeuchtigkeit vorhanden ist oder kleinste Undichtigkeiten bestehen. Ebenso können ggf. weitere Bauelemente in die Hülse eingesetzt werden.

Weiterhin ist es bevorzugt, dass der Zwischenring durch Schweißen in die Hülse des Endoskops gefügt wird. Hierdurch wird eine dauerhaft feste und dampfdichte Verbindung erzielt. Durch Schweißen wird der weitere Vorteil erreicht, dass etwaige Spannungen, die vom Zwischenring auf das Deckglas ausgeübt werden, weiter abgebaut werden. Es können jedoch auch andere Fügetechniken zur Verbindung des Zwischenrings mit der Hülse eingesetzt werden, beispielsweise Löten oder Kleben.

Gemäß einer bevorzugten Ausführungsform des erfindungsgemäßen Verfahrens ist die Hülse eine Okularmuschelhülse, die eine Okularmuschel trägt und den proximalen Teil des optischen Systems des Endoskops aufnimmt. Das Deckglas dient in diesem Fall als proximales Endfenster des Endoskops.

In bevorzugter Weise kann die Okularmuschel auf die Okularmuschelhülse aufgeschraubt werden. Dadurch, dass eine vorgefertigte Okularmuschel auf die Hülse aufgeschraubt wird, können Fehler durch Achsabweichungen ebenso wie Taumelfehler weitgehend vermieden werden; solche Fehler können sich insbesondere dann nachteilig bemerkbar machen, wenn an die Okularmuschel ein elektronischer Bildaufnehmer, beispielsweise eine Videokamera, angesetzt wird, da für diese eine möglichst exakt axiale Anordnung wünschenswert ist.

Ein erfindungsgemäßes Endoskop weist ein langerstrecktes Rohr auf, das insbesondere starr oder semiflexibel ausgebildet sein kann, in dessen Innerem mindestens ein Teil eines optischen Systems zum Weiterleiten eines endoskopischen Bildes von einem distalen zu einem proximalen Ende des Endoskops angeordnet ist. Das optische System ist am distalen und/oder proximalen Ende durch ein Deckglas abgeschlossen, wobei das Deckglas ohne eine dazwischenliegende Beschichtung in einen Zwischenring eingelötet ist, der in eine mit dem Rohr verbundene oder das Rohr bildende Hülse des Endoskops gefügt ist, und wobei das Deckglas mit einer Antireflexbeschichtung versehen ist. Die Verbindungen zwischen dem Deckglas und dem Zwischenring sowie zwischen dem Zwischenring und der Hülse bzw. dem Rohr sind insbesondere dampfdicht ausgeführt. Das optische System des Endoskops ist hierdurch bevorzugt dampfdicht oder hermetisch dicht abgeschlossen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 einen Längsschnitt durch den proximalen Endbereich eines Endoskops gemäß einem Ausführungsbeispiel der Erfindung;
Fig. 2 ein vergrößertes Detail des proximalen Endes des Endoskops der Fig. 1, ebenfalls im Längsschnitt.

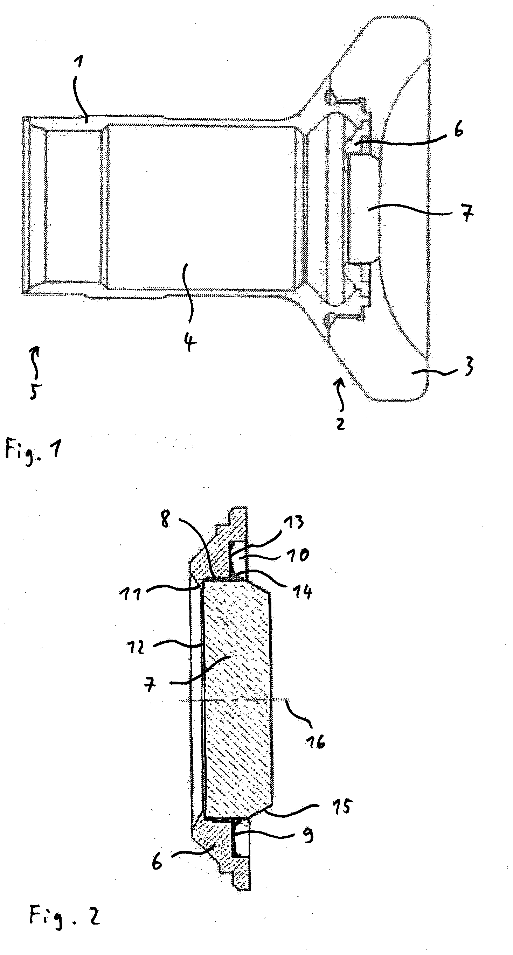

Gemäß Fig. 1 umfasst der proximale Endbereich eines Endoskops eine Okularmuschelhülse 1, auf deren proximales Ende 2 eine Okularmuschel 3 aufgeschraubt ist. Die Okularmuschel kann aus Kunststoff, beispielsweise Polyetheretherketon (PEEK), bestehen und zusätzlich durch Kleben mit der Okularmuschelhülse 1 verbunden sein. Die Okularmuschelhülse weist einen Innenraum 4 auf, der einen proximalen Endbereich des optischen Systems des Endoskops enthält, der Linsen oder andere optische Elemente umfassen kann, die in einem Innenrohr aufgenommen sein können (nicht dargestellt). Die Okularmuschelhülse 1 geht an ihrem distalen Ende 5 in ein Außenrohr über, das das optische System umschließt, oder ist mit diesem verbunden (nicht dargestellt); das Außenrohr kann den Endoskopschaft bilden. Insbesondere ist die Okularmuschelhülse 1 dampfdicht mit dem Außenrohr verbunden. Ein ggf. vorhandenes Innenrohr kann zusätzlich dampfdicht ausgebildet sein. Die Okularmuschelhülse 1 ist bei einem typischen Durchmesse des Deckglases von ca. 1 cm üblicherweise einige Zentimeter lang, beispielsweise ca. 3 cm.

Im Bereich des proximalen Endes 2 der Okularmuschelhülse 1 ist in diese ein Zwischenring 6 eingefügt, der als Stahlring ausgebildet ist. Der Zwischenring 6 trägt ein Deckglas 7, das hier als proximales Endfenster des Endoskops dient und eine visuelle Betrachtung des vom optischen System weitergeleiteten endoskopischen Bildes ermöglicht. An die Okularmuschel 3 kann eine Videokamera angesetzt werden, wobei die Optik der Videokamera die aus dem Deckglas austretenden Strahlen auf einen elektronischen Bildaufnehmer fokussiert (nicht dargestellt). Das Deckglas ist im Ausführungsbeispiel als Planglas ausgebildet, könnte aber gekrümmte Flächen aufweisen, die eine optische Wirkung haben.

Wie in der vergrößerten Detaildarstellung der Fig. 2 zu erkennen, ist zwischen einer zylindrischen Innenwand des Zwischenrings 6 und einer ebenfalls zylindrischen Umfangsfläche des Deckglases 7 ein umlaufender Spalt 8 ausgebildet. Zur Verbindung des Deckglases 7 mit dem Zwischenring 6 wird beim Aktivlöten das Lot 9 in die durch das Positionieren des Deckglases 7 im Zwischenring 6 entstandene umlaufende Nut 10 eingebracht. Das flüssige Lot 9 dringt aufgrund von Kapillarkräften in den Spalt 8 ein und füllt diesen weitgehend aus, wobei auch ein eventueller Spalt zwischen einem radial nach innen weisenden ringförmigen Vorsprung 11 des Zwischenrings 6 und der bezüglich des Endoskops inneren Oberfläche 12 des Deckglases durch das Lot gefüllt werden kann. Die den Boden der Nut bildende ringförmige Planfläche 13 des Zwischenrings 6 kann ebenfalls mit Lot 9 benetzt sein.

Durch die Füllung des Spalts 8 mit Lot 9 wird eine höchstmögliche Sicherheit gegen Undichtigkeiten erreicht, weil eventuell verbleibende Poren vom Lot umschlossen sind und daher keine Undichtigkeit verursachen können. Hierfür hat der Spalt 8 eine ausreichende Höhe, die beispielsweise ca. 1 mm betragen kann. Die zylindrische Umfangsfläche des Deckglases 7 ragt in axialer Richtung über die zylindrische Innenwand des Zwischenrings 6 hinaus. Zwischen der Umfangsfläche des Deckglases 7 und der ringförmigen Planfläche 13 bildet das verbleibende Lot eine Kehlnaht 14, die zusätzlich zur Dichtheit und zur Sicherheit der Verbindung beiträgt. Außerhalb der zylindrischen Umfangsfläche weist das Deckglas 7 eine umlaufende Fase 15 auf. Die optische Achse des Deckglases 7 bzw. des optischen Systems des Endoskops ist in Fig. 2 mit dem Bezugszeichen 16 gekennzeichnet. Zur Montage des Deckglases 7 in einem Endoskop wird zunächst das Deckglas 7 in den Zwischenring 6 gegen den radialen Vorsprung 11 angelegt. Sodann wird das Deckglas 7 wie vorstehend ausgeführt durch Aktivlöten mit dem Zwischenring 6 dampfdicht verbunden. Sodann wird auf den freien Teil der inneren Oberfläche 11 eine Antireflexbeschichtung aufgebracht; dieser Vorgang wird durch den Zwischenring 6 nicht wesentlich eingeschränkt. Ein dabei ggf. erfolgendes Aufbringen der Beschichtung auf Teilbereiche des Zwischenrings 6 schränkt dessen Funktion nicht ein. Nach dem Einbringen von Trockenmittel in die Okularmuschelhülse 1 wird die Einheit aus Zwischenring 6 und Deckglas 7 in die Okularmuschelhülse 1 eingefügt, indem der Zwischenring 6 in den proximalen Endbereich der Okularmuschelhülse 1 eingelegt und mit einem Fügeverfahren in diesen dampfdicht gefügt wird, insbesondere mit ihm verschweißt wird. Schließlich wird die Okularmuschel 3 auf die Okularhülse 1 aufgeschraubt. Die Okularmuschel 3 übergreift dabei das Deckglas 7 an der Fase 15, so dass dieses zusätzlich gegen Herausfallen gesichert ist.

### Bezugszeichenliste

- 1: Okularmuschelhülse
- 2: Proximales Ende
- 3: Okularmuschel
- 4: Innenraum
- 5: Distales Ende
- 6: Zwischenring
- 7: Deckglas
- 8: Spalt
- 9: Lot
- 10: Nut
- 11: Vorsprung
- 12: Oberfläche
- 13: Planfläche
- 14: Kehlnaht
- 15: Fase
- 16: Optische Achse

## Patentansprüche

1. Verfahren zum Montieren eines Deckglases (7) in einem Endoskop, wobei ein unbeschichtetes Deckglas (7) in einen Zwischenring (6) eingelötet wird, das in den Zwischenring (6) eingelötete Deckglas (7) zumindest auf einer Innenseite (12) mit einer Beschichtung versehen wird, ein Trockenmittel in eine Hülse des optischen System des Endoskops eingebracht wird und der mit dem beschichteten Deckglas (7) versehene Zwischenring (6) in die Hülse des optischen Systems des Endoskops gefügt wird, wobei die Hülse eine Okularmuschelhülse (1) ist und das Deckglas (7) ein proximales Endfenster des Endoskops ist.

2. Verfahren nach Anspruch 1, **dadurch gekennzeichnet, dass** das Deckglas (7) durch Hartlöten, insbesondere durch Aktivlöten, in den Zwischenring (6) eingelötet wird.

3. Verfahren nach Anspruch 1 oder 2, **dadurch gekennzeichnet, dass** das Deckglas (7) dadurch in den Zwischenring (6) eingelötet wird, dass das Deckglas (7) in den Zwischenring (6) eingesetzt wird, wobei ein Spalt (8) zwischen einer Umfangsfläche des Deckglases (7) und einer Innenfläche des Zwischenrings (6) verbleibt, und dass beim Löten das Lot (9) in den Spalt (8) eindringt und diesen zumindest teilweise ausfüllt.

4. Verfahren nach einem der vorhergehenden Ansprüche, **dadurch gekennzeichnet, dass** das Lot (9) zwischen einer Umfangsfläche des Deckglases (7) und einer ringförmigen Planfläche des Zwischenrings (6) eine Kehlnaht (12) bildet.

5. Verfahren nach einem der vorhergehenden Ansprüche, **dadurch gekennzeichnet, dass** die Beschichtung eine Antireflexschicht ist.

6. Verfahren nach einem der vorhergehenden Ansprüche, **dadurch gekennzeichnet, dass** der Zwischenring (6) durch Schweißen in die Hülse des Endoskops gefügt wird.

7. Verfahren nach einem der vorhergehenden Ansprüche, **dadurch gekennzeichnet, dass** eine Okularmuschel (3) auf die Okularmuschelhülse (1) aufgeschraubt wird.

## Claims

1. A method for assembling a cover glass (7) in an endoscope, wherein an uncoated cover glass (7) is soldered into an intermediate ring (6), the cover glass (7) soldered into the intermediate ring (6) is provided with a coating, at least on an inner side (12), a drying agent is introduced into a sheath of the optical system of the endoscope and the intermediate ring (6) provided with the coated cover glass (7) is joined into the sheath of the optical system of the endoscope, wherein the sheath is an eyecup sheath (1) and the cover glass (7) is a proximal end window of the endoscope.

2. The method according to claim 1, **characterized in that** the cover glass (7) is soldered into the intermediate ring (6) by hard soldering, in particular by active soldering.

3. The method according to claim 1 or 2, **characterized in that** the cover glass (7) is soldered into the intermediate ring (6) **in that** the cover glass (7) is inserted into the intermediate ring (6), wherein a gap (8) remains between a surrounding surface of the cover glass (7) and an inner surface of the intermediate ring (6), and **in that** the solder (9) penetrates into the gap (8) upon soldering and at least partly fills it.

4. The method according to any one of the preceding claims, **characterized in that** the solder (9) forms a fillet weld (12) between a surrounding surface of the cover glass (7) and a ring-shaped plane surface of the intermediate ring (6).

5. The method according to any one of the preceding claims, **characterized in that** the coating is an anti-reflecting coating.

6. The method according to any one of the preceding claims, **characterized in that** the intermediate ring (6) is joined into the sheath of the endoscope by welding.

7. The method according to any one of the preceding claims, **characterized in that** an eyecup (3) is screwed onto the eyecup sheath (1).

## Revendications

1. Procédé de montage d'une lamelle couvre-objet (7) dans un endoscope, dans lequel une lamelle couvre-objet (7) non revêtue est brasée dans un anneau intermédiaire (6), dans lequel la lamelle couvre-objet (7) brasée dans l'anneau intermédiaire (6) est pourvue d'un revêtement, au moins sur une face intérieure (12), dans lequel un agent de séchage est introduit dans une douille du système optique de l'endoscope et dans lequel l'anneau intermédiaire (6) pourvu de la lamelle couvre-objet (7) revêtue est inséré dans la douille du système optique de l'endoscope, dans lequel la douille est une douille de bonnette d'oculaire (1) et la lamelle couvre-objet (7) est une fenêtre d'extrémité proximale de l'endoscope.

2. Procédé selon la revendication 1, **caractérisé en ce que** la lamelle couvre-objet (7) est brasée dans l'anneau intermédiaire (6) par brasage fort, notamment par brasage actif.

3. Procédé selon la revendication 1 ou la revendication 2, **caractérisé en ce que** la lamelle couvre-objet (7) est brasée dans l'anneau intermédiaire (6) **en ce que** la lamelle couvre-objet (7) est insérée dans l'anneau intermédiaire (6), dans lequel il reste une fente (8) entre une surface de circonférence de la lamelle couvre-objet (7) et une surface intérieure de l'anneau intermédiaire (6), et que, pendant le brasage, la brasure (9) pénètre dans l'espace et au moins partiellement remplit cette fente (8).

4. Procédé selon l'une quelconque des revendications précédentes, **caractérisé en ce que** la brasure (9) forme une soudure d'angle (12) entre une surface de circonférence de la lamelle couvre-objet (7) et une surface plane annulaire de l'anneau intermédiaire (6).

5. Procédé selon l'une quelconque des revendications précédentes, **caractérisé en ce que** le revêtement est une couche anti-reflet.

6. Procédé selon l'une quelconque des revendications précédentes, **caractérisé en ce que** l'anneau intermédiaire (6) est inséré dans la douille de l'endoscope par soudage.

7. Procédé selon l'une quelconque des revendications précédentes, **caractérisé en ce qu'**une bonnette d'oculaire (3) est vissée sur la douille de bonnette d'oculaire (1).
